# EUROPEAN PATENT SPECIFICATION

(11) **EP 2 904 373 B1**
(45) Date of publication and mention of the grant of the patent: **14.12.2022**
(21) Application number: 13802437.7
(22) Date of filing: 25.09.2013
(51) Int. Cl.: G01N 1/28

(54) **COMBINED SAMPLE EXAMINATIONS**
KOMBINIERTE PROBENUNTERSUCHUNGEN
EXAMENS D'ÉCHANTILLONS COMBINÉS

(30) Priority: 03.10.2012 US 201261709243 P
(43) Date of publication of application: 12.08.2015
(73) Proprietor: Koninklijke Philips N.V., 5656 AG Eindhoven (NL)
(72) Inventor: VINK, Jelte Peter, NL-5656 AE Eindhoven (NL); WIMBERGER-FRIEDL, Reinhold, NL-5656 AE Eindhoven (NL); VAN DE STOLPE, Anja, NL-5656 AE Eindhoven (NL)
(74) Representative: Philips Intellectual Property & Standards
(86) International application number: PCT/IB2013/058847
(87) International publication number: WO 2014/053955

(56) References cited:
- EP-A1- 1 953 662
- WO-A2-01/33190
- US-A- 6 159 681
- US-A1- 2010 020 299
- US-A1- 2010 081 923
- US-A1- 2011 177 518
- US-B1- 6 194 157
- HADANO S ET AL: "Laser microdissection and single unique primer PCR allow generation of regional chromosome DNA clones from a single human chromosome", GENOMICS, ACADEMIC PRESS, SAN DIEGO, US, vol. 11, no. 2, 1 October 1991 (1991-10-01), pages 364-373, XP024865131, ISSN: 0888-7543 [retrieved on 1991-10-01]

## Description

### FIELD OF THE INVENTION

The invention relates to a method and an apparatus for the examination of a sample, particularly of a sample of biological tissue. Moreover, it relates to a sample isolation unit.

### BACKGROUND OF THE INVENTION

The US 6 091 842 discloses an analyzer in which images of a biological specimen are generated and automatically analyzed for regions containing cytological material. These regions are then automatically presented to a human operator in a temporarily optimized sequence.

The US 6 159 681 discloses a method for performing regional analysis of biologic materials. The method employs a photoresist layer that is established over a biologic material. Regions of interest are selected and irradiated to expose specific regions of biologic material. Exposed biologic material may then be selectively analyzed using any of a variety of analytic methods.

Hadano S ET AL: "Laser microdissection and single unique primer PCR allow generation of regional chromosome DNA clones from a single human chromosome", Genomics, Academic Press, San Diego, US, vol. 11, no. 2, 1 October 1991, pages 364-373, discloses the development of an argon laser chromosome microdissection technique in conjunction with a polymerase chain reaction (PCR) approach to directly amplify microdissected chromosomes.

US 2011/177518 discloses devices and methods for the micro-isolation of biological cellular material. The micro-isolation devices and methods utilize a photomask that protects regions of interest against DNA-destroying illumination.

WO 01/33190 discloses systems and methods for automated laser capture microdissection, high throughput microdissection is provided by using cell procurement and multi-imaging tools for pre-selection cells of interest.

US 6 194 157 discloses a method for separating biological substances by using photoresist. After a photoresist is used to fix a biological sample, in order to obtain a particular biological substance, for example, a certain cell or biopolymer, from the biological sample embedded in the photoresist, properties of the photoresist are changed by exposing the portion of the photoresist that covers the biological substance to light L which has an appropriate wavelength. The biological substance embedded in the changed photoresist is collected.

US 2010/081923 discloses systems, methods and other modalities for generating or otherwise handling images or other data indicating (a) an extraction of chemically treated tissue frozen in vivo, (b) a treatment of a tissue sample in a chamber extended into tissue of an organism, and/or (c) cells to which an optical enhancement material was applied in vivo.

EP 1 953 662 A1 provides a system to perform a molecular histological analysis of a multicellular sample based on a modified digital image.

### SUMMARY OF THE INVENTION

It is an object of the invention to provide means for a more versatile examination of a sample. It is desirable that this examination has a high accuracy and/or efficiency.

This object is addressed by a method according to claim 1, and an apparatus according to claim 2.

Preferred embodiments are disclosed in the dependent claims.

According to its first aspect, the invention addresses the above concerns by a method for the examination of a sample, particularly a sample of biological origin like a piece of tissue that shall be examined e.g. for the presence of tumor cells. The method comprises at least one of the following steps or a combination thereof, wherein steps or sequences of steps can optionally be repeated one or more times:
a) The generation of an image of the sample.
b) The analysis of said image with respect to at least one given parameter that can be determined from the image and that will be called "sample-parameter" in the following for purposes of reference. Typically, the sample-parameter will be determined in a spatially resolved way, i.e. in dependence on image regions or image-pixels. Reference to "the sample-parameter" will in the following mean a reference to at least one sample-parameter, preferably to all sample-parameters, if more than one type of sample-parameter has been determined.
c) The selection of a region of interest (ROI) in the mentioned image, wherein said region of interest will be called "image-ROI" in the following.
d) The isolation of a region from the sample that corresponds to the aforementioned image-ROI, wherein this region of the sample will in the following be called "sample-ROI". The "isolation" means that sample material belonging to the sample-ROI is physically separated from the remainder of the sample.
e) The execution of at least one molecular assay with the aforementioned sample-ROI.
f) The linking of assay-data to the sample-parameter of the corresponding sample region.

The abovementioned image-ROI may be a single connected patch of the image, or it may consist of a plurality of disconnected patches. The decision if a point of the image belongs to the image-ROI or not depends on the intended examination of the sample. In oncology applications, the image-ROI may for example contain those image portions that are suspected to show tumor cells.

The correspondence between image-ROI and sample-ROI is usually such that
a) each point of the image that belongs to the image-ROI shows a position of the sample that belongs to the sample-ROI and/or
b) each point of the sample that belongs to the sample-ROI is represented by a point of the image that belongs to the image-ROI.

Typically, both relations a) and b) hold simultaneously (bijective relation), but it is also comprised by the invention that only a) or only b) holds (injective relation).

Moreover, the term "molecular assay" is to be understood in a broad sense, comprising any examination, test, or experiment by which one or more parameters of the sample-ROI that depend on its chemical composition may be determined. As an example, the molecular assay may comprise the (qualitative or quantitative) detection of particular proteins or nucleic acid sequences (e.g. tumor markers).

The linking in step f) means that assay-data determined in the molecular assay(s) for the sample-ROI and values of the sample-parameter determined for the corresponding sample region - i.e. the image-ROI - are associated to each other. This may for example be done by storing them together at a given location in a memory, database, or table, or by referring to them with a pointer. Due to the linkage, assay-data can then be evaluated automatically and/or by a user taking into account the sample-parameter belonging to the same part of the sample (or vice versa).

According to a second aspect, the invention relates to an apparatus for the examination of a sample, said apparatus comprising at least one of the following components or a combination thereof:
a) An image generating unit for the generation of an image of the sample.
b) An image analyzer for analyzing said image with respect to at least one sample-parameter.
c) An image selection unit for the selection of a region of interest (ROI) in the aforementioned image, said region being called "image-ROI" in the following.
d) A sample isolation unit for the isolation of a region of interest from the sample that corresponds to the aforementioned image-ROI in the image and that is called "sample-ROI" in the following.
e) A molecular examination unit for the execution of a molecular assay with the sample-ROI.
f) An evaluation unit for linking assay-data to the sample-parameter of the corresponding sample region. The functions of the image analyzer, of the image selection unit, and/or of the evaluation unit may optionally be provided by the same device, for example by a general purpose computer.

The method and the apparatus according to the first and second aspects of the invention comprise the same inventive concept that a region of interest in a sample is first selected from an image of the sample and then extracted in reality from the physical sample and subjected to a molecular assay, wherein corresponding image-data and assay-data are linked to each other. In particular, the method can be executed with the described apparatus. Explanations and definitions provided for the method are therefore also valid for the apparatus and vice versa.

The method and the apparatus have the advantage that they allow for a more informative examination of a sample because a specimen can be subjected both to visual inspection and to molecular diagnostics, wherein the results are linked to each other in a spatially resolved way. Moreover, a high precision of the molecular assay can be achieved because the inclusion of non-relevant (disturbing) sample material is minimized by the targeted definition of the sample-ROI and because visual inspection and molecular assay refer to the very same material (and not e.g. to different slices of a sample). A further advantage is that many or even all of the processing steps can be automated, thus achieving a high efficiency of the whole examination procedure.

The analysis of sample material for molecular diagnostics ("MDx") analysis can derive parameters that are either critical to the quality of the MDx analysis or provide additional information that enables a better interpretation of the MDx results. For example, when selection is based on individual cells the secure identification of those cells is required. This can be for instance tumor cells, characterized by parameters, like a certain ratio between nucleus and cytoplasm, a certain shape irregularity of the nucleus, a particular immunestaining of proteins in the cell membrane (e.g. HER2) or the cytoplasm (cytokeratin), nuclear receptors (like ER), or genomic parameters, like the copy number of certain genes (e.g. Her2) or the copy numbers of mRNAs, or combinations thereof. Certain complementary stains can be used to rule out false calls by identifying cells that have tumor like appearance with specific immune-stains for epithelial, stromal or immune cells, etc.

Another example is the usage of image parameters (sample-parameters) for the interpretation of MDx results. MDx tests, carried out with methods like q-PCR, micro-arrays, Sanger sequencing and Next Gen sequencing have certain sensitivity and specificity limitations. In addition when looking at gene expression patterns, the particular profile will depend on the type of cells that is analyzed. Using the information from the analysis of the image and/or the image-ROI can enable a more precise interpretation of the expression profile. Expression can for example be scaled to the fraction of tumor cells and corrected for contributions from nominal expression profiles of other cell types (e.g. nonmalignant). Sequencing data can for example be corrected for reference genomic contributions stemming from the non-tumor cells according to the fractional presence in the ROI. In certain cases when the results of the MDx test are ambiguous the role of sample selection can be taken into account and another more stringent selection can be advised. The sample can be revisited for identifying alternative ROIs based on the conclusions from the first MDx test.

In the following, various preferred embodiments of the invention will be described that can be realized with both the method and the apparatus.

The sample to be examined may particularly be a slice of body tissue. Moreover, the sample may be stained before the image is generated in order to make particular features of interest (better) visible. Accordingly, the method of the invention may optionally comprise as first steps the generation of a slice of body tissue and/or the staining of the sample. In the apparatus of the invention, a sample preparation unit may optionally be included in which these steps can be executed.

Staining can additionally or alternatively be done at other times during the whole examination of the sample. In some embodiments of the invention, the results of the MDx test can for example be used to choose a follow up staining assay on a sample section. That staining can optionally be carried out on the remaining sample section that has already been analyzed and partially removed. In this case the previous results can be included (e.g. on individual cell bases) with the new results obtained from the follow up staining. As an example, the MDx assay can provide insights about the genetic mutations and/or the activity of a certain signaling pathway in the tumor cells. A particular staining for presence and/or activation of proteins that play a role in that pathway can provide important information about the relation between the genetic information and tumor progression and/or susceptibility to certain treatment regimens.

The generated image of the sample is preferably a microscopic image, i.e. it reveals details not visible to the naked eye. Additionally or alternatively, it is preferably a digital image, thus allowing for the application of versatile digital image processing procedures. Furthermore, the image may be generated by scanning, i.e. by the sequential generation of sub-images of smaller parts of the sample. The apparatus may accordingly comprise a digital microscope, particularly a digital scanning microscope, to allow for the embodiment of the aforementioned features. Furthermore, the generated microscopic image can be a brightfield or fluorescence image, or a combination of different images.

The sample is preferably covered by a cover slip during the generation of the image. This is for example desirable when a digital microscope is used, particularly a whole slide scanner. Image analysis from whole slide scanners requires a high image quality, since the pathologist needs to be able to extract all information he/she would otherwise get from manipulation with a microscope. High image quality requires samples to be embedded covered, a standard procedure in pathology labs, called cover slipping. Commercial equipment is available for that step. Since with digital pathology the digital file can be stored and archived instead of the stained slide, the slide can be used for retrieval of the sample for MDx. This has the advantage of having a 100% match between the analyzed tissue and cells and the selected material for MDx, while otherwise projections and interpolations are required to relate the image to the next section from the sample (paraffin block) from which the ROI for MDx is dissected.

Having the possibility to take the sample from the identical coupe from which the digital file has been recorded, however, requires an extra sample preparation step. For the removal of the ROI the cover slip is an obstacle. It is therefore preferably removed before isolation of the sample-ROI. The slide can be physically aligned to the same reference (marker) as for digital scanning. No new image is required to make the physical sample selection. The virtual image can be used to guide the laser beam, or mechanical, or other device that captures the sample-ROI and removes it from the remaining sample. Optionally, lower quality images can be obtained in that state on the selection apparatus and mapped to the high quality stored images to help alignment and selection.

Once the cover slip has been removed, additional stainings can be carried out on the same slide before and/or after the removal of the sample-ROI for reasons described above. After coverslipping high quality images can be obtained that are analyzed and interpreted together with the results of all previous tests, most importantly the MDx tests.

The selection of the image-ROI may be done automatically by appropriate image processing routines, by the manual input of a user, or by a mixture of both. Accordingly, the apparatus may preferably comprise an image analysis module, for example a digital microprocessor with associated software for the analysis of digital images. Additionally or alternatively it may comprise a user interface comprising input means by which a user can input data referring to the selection of an image-ROI. Typically, the user interface will also comprise output means, for example a display (monitor) on which the image of the sample can be shown, optionally together with a representation of the currently defined image-ROI. The output means may preferably allow for a representation of the sample image with adjustable zooming factor.

The image analyzer will typically be a digital data processing unit with appropriate image processing software by which the sample-parameter can be determined automatically.

The sample-parameter may in general be any type of parameter that can be determined from the image of the sample, for example the local concentration of a given chemical substance (revealed e.g. via the color of the substance). In the invention, the sample-parameter indicates the local amount of a particular cell-type or tissue-type. The sample-parameter may for instance express the absolute or relative number of tumor cells in a given region. In particular, it may be the number and/or fraction of tumor cells in the image-ROI. Knowing this number for the image-ROI may provide important clues for a correct interpretation of the assay-data that refer to this region.

Another advantageous embodiment of the invention is achieved if the sample-parameter is (at least partially) based on a staining assay executed with the sample. Possible staining assays include for example H&E (Hematoxylin-Eosin) for morphology, IHC (immuno-histochemistry), FISH (fluorescence in situ hybridization), PLA (proximity ligation assay, from Olink, Sweden), PPA (padlock probe assay, from Olink, Sweden), rolling circle amplification, RCA (Olink, Sweden), branched DNA signal amplification, and combinations of all these techniques or other assays to obtain particular biological information. The staining may especially help to identify particular cell types or tissue types, and/or molecules which indicate a specific property or function or abnormality of the cell.

The selection of the image-ROI is based on the determined sample-parameter. If the sample-parameter indicates for example the local amount of tumor cells, the image-ROI may be chosen to comprise those regions in which this parameter is above a given threshold.

While it is usually possible to generate an image-ROI of nearly arbitrary shape and size, this will typically not be the case for the sample-ROI because this has to be realized with the actual physical sample. In a preferred embodiment of the invention, the size and/or shape of the image-ROI is therefore adapted according to requirements set by the possible sample-ROIs. For example, the size of the image-ROI and/or the curvature of its border may be restricted to be larger than a given minimum or smaller than a given maximum, respectively. The adaptation has the advantage that only such image-ROIs are generated that can actually be transferred into a physical sample-ROI, thus avoiding a mismatch between the intended and the actual selection of sample for the molecular assay. The adaptation may preferably be done automatically by appropriate (digital) image processing routines, for example based on a given user selection.

Obtaining a pure fraction of cells of interest can be time consuming and requires a high definition with removal. The aforementioned approach allows to relax the requirements for selection and balance that with additional parameters that take into account how easy or reliable sections can be removed and control the total size of the selection. For a convenient selection larger, continuous sections are preferred. The image analysis can provide tabular parameters, like the number and fractions of each identified cell type in a potential area of interest, e.g. the total surface area. The shape of the area can be limited by design criteria including parameters, like total area, allowable curvatures and connectivity. Based on an algorithm an optimum can be determined given a certain selection algorithm which can be specific for each MDx assay. Rather than providing a homogenous sample, a well characterized sample is obtained in this way that fulfills requirements with respect to the MDx test, like the fraction of tumor cells and requirements for easy isolation, like the geometrical parameters of the selected ROIs.

There are several possibilities how a sample-ROI can be isolated from the sample. One possibility is the application of laser microdissection that is known from literature (cf. Falko Fend, Mark Raffeld: "Laser capture microdissection in pathology", J. Clin. Pathol. 2000, 53:666-672). Another possibility is the usage of a printing device with which an indication of the sample-ROI can be printed onto the sample itself, thus allowing a human operator (or a machine) to separate the sample-ROI from the remainder of the sample. According to a further embodiment, the image-ROI can be represented in a (e.g. digital) microscope so that it can be seen by a human operator or a machine together with the original sample.

The sample may preferably be provided on some carrier to allow for an easy handling. For example, a slice of body tissue will typically be provided on a microscope slide as carrier. Typically materials of the carrier (or substrate) on which a sample may be provided comprise glass, transparent plastic, and/or composites of glass and plastic, optionally with a surface layer for the desired interaction with the biological sample. Moreover, the carrier may have the form of a cartridge, for example a cartridge with an open cavity, a closed cavity, or a cavity connected to other cavities by fluid connection channels.

The aforementioned carrier may preferably comprise at least one marker, i.e. an element on the carrier which can readily be localized in reality and in the image of the sample. The marker therefore provides a reference that allows to map an "image-coordinate system" (in the image plane) onto a "sample-coordinate system" (referring to the actual sample on the carrier). This mapping of coordinates is important for the proper isolation of the sample-ROI, which has to be done in physical space based on image coordinates.

When the sample is provided on a carrier, the sample-ROI is preferably transferred from said carrier to a separate holder (a container, cartridge, tube etc.) after or during its isolation from the remainder of the sample. The separate holder can then further be transferred to the molecular examination device for executing the desired molecular assays with the sample-ROI. The remainder of the sample, on the contrary, may remain on the carrier and for example be stored or discarded.

According to a further development of the invention, an image of the sample-ROI and/or of the remaining sample is generated after the isolation of the sample-ROI. This image may be generated with the same image generating unit that also generated the image of the whole sample, or with separate device. The image of the sample-ROI (or of the remainder of the sample) can be compared to the image of the whole sample and particularly to the selected image-ROI, thus allowing for a verification if the actual sample-ROI corresponds to the desired region of interest or not.

It was already mentioned that the molecular assay may comprise one or more of a large variety of different tests. In particular, the molecular assay may comprise PCR (e.g. q-PCR, qRT-PCR, RT-PCR, qrt-PCR, or digital PCR), sequencing (particularly next gen sequencing), or micro-array hybridization, or another molecular assay technology or a combination of these.

The image-data showing the whole sample (and optionally also the selected image-ROI) and the assay-data that were generated during the molecular assay(s) are preferably combined in such a way that they are simultaneously accessible to a user. The specific tissue staining data and molecular assay data can be interpreted to provide additional information on individual cellular function or characteristics. Accordingly, the apparatus preferably comprises a user interface at which image-data and assay-data are accessible. The user interface may for example comprise a memory for the data and a display (monitor) on which the data can simultaneously be displayed, preferably in such a way that the assay-data are shown at the image location from which they originate. Thus the collected information can be presented in a user-friendly and intuitive way, facilitating their evaluation.

According to another development of the invention, a plurality of sample-ROIs is isolated (based on a corresponding plurality of image-ROIs), wherein different sample-ROIs are subjected to individually different assays. Thus it will be possible to investigate in the same sample different regions with respect to different questions, searching for example for a first marker in one region and for another marker in another region.

The aforementioned definition and isolation of image-ROIs and sample-ROIs may take place in parallel (simultaneously) and/or sequentially. Selection of a (second) image-ROI and isolation of a corresponding (second) sample-ROI may for example be done based on the combined results of the previous examination of a (first) image-ROI and a corresponding (first) sample-ROI. Such a follow up of examinations may be repeated even more than one times.

According to a third aspect, the invention relates to a sample isolation unit for isolating a sample-ROI in a sample of biological origin like a piece of tissue that shall be examined e.g. for the presence of tumor cells, wherein said sample isolation unit comprises the following components:
- A light source for generating a light beam.
- A light directing system for directing the aforementioned light beam to positions outside the sample-ROI such that the sample material at these positions is altered to become separable from the remainder of the sample.

The described sample isolation unit can particularly be used in an apparatus of the kind described above. It has the advantage that it allows for a simple and versatile isolation of a region of interest in a sample by treating the part of the sample not belonging to this region appropriately with a light beam. Due to the flexibility and precision with which a light beam can be controlled, it is thus possible to isolate a sample-ROI of nearly any arbitrary shape.

According to a fourth aspect, the invention relates to a method for the isolation of a sample-ROI from a biological sample, said method comprising the following steps:
- Generating a light beam.
- Directing the aforementioned light beam to positions outside the sample-ROI such that the sample material there is altered to become separable from the remainder.

The sample isolation unit and the method described above are based on the same inventive concept, i.e. the treatment of regions of a sample outside a sample-ROI by a light beam. Explanations and definitions provided for the sample isolation unit are therefore also valid for the method and vice versa. In the following, various preferred embodiments of the invention will be described that relate both to the sample isolation unit and the method.

Preferably, all positions outside the sample-ROI are treated in the described way. With other words, the whole complement of the sample-ROI is treated.

In general, any reaction of the sample material to the light beam by which said material becomes separable from the sample-ROI (i.e. from untreated sample material) is comprised by the present invention. For example, the sample material may be fixed ("burned-in") to a carrier on which the sample is provided, thus allowing a later selective removal of the untreated sample-ROI from said carrier.

In a preferred embodiment of the invention, the alteration of the sample material outside the sample-ROI by the light beam comprises the ablation of sample material. Thus the undesired sample material is simply removed, leaving the desired sample-ROI behind. An advantage of this approach is that ablation of the sample material is particularly possible with nearly all kinds of biological tissue provided that sufficient light energy is applied to the respective regions. Moreover, no transfer of the sample-ROI to another container or holder is necessary, which further simplifies the workflow.

According to a further development of the aforementioned approach, a waste depot may be provided for collecting ablated sample material. The waste depot may preferably be exchangeable such that it can be renewed from time to time, for example after each isolation of a sample-ROI. Providing a waste depot avoids problems with an uncontrolled deposition of ablated sample material, which might for example lead to a contamination of the sample-ROI.

In order to enable and/or support the alteration of the sample material by the light beam, the sample material may comprise a light-sensitive reagent that has been added before the light treatment. The reagent may for example be a staining agent that is used for microscopic investigations and to which a molecule or chemical agent is coupled that can be activated by light to destroy the stained area.

The light beam used for the alteration of sample material outside the sample-ROI may particularly comprise a high power LED light beam or a laser light beam, which has the advantage to allow for a spatially well localized application of high intensities.

In typical cases, the power density of the applied light beam is higher than about 0.1 mW/µm², preferably higher than about 1.0 mW/µm². When a modulated light beam is applied, for example a pulsed laser, the aforementioned values refer to the mean power density that is determined over the whole period of light application.

In general, the light of the light beam will favorably have a spectrum comprising wavelengths that are well absorbed by biological tissue. Such wavelengths may typically comprise UV (ultraviolet) light (about 100 nm to 380 nm) and/or IR (infrared) light (about 800 nm to about 1 mm), but also visible light. IR light is particularly suited for an ablation of (biological) sample material.

In one embodiment, the light beam may be adapted to illuminate the complete sample at hand simultaneously. In another embodiment, the light directing system may comprise a scanning element (e.g. a movable mirror and/or a movable sample-holder) for scanning the light beam across the sample or a part thereof. In this case the light beam reaches only small, limited parts of the sample at a time, wherein these parts are sequentially and repetitively moved over the whole sample or a part thereof.

In each of the aforementioned embodiments, care must be taken that the light beam does not (or at least not with an intensity above a given threshold) reach positions in the sample-ROI. This can be achieved if the light directing system comprises a light-control element for (completely or partially) suppressing the generation and/or the propagation of the light beam if it would reach positions within the sample-ROI.

In case of the abovementioned scanning of the light beam, the light-control element may control a scanning element of the light directing system such that it directs the light beam only to the desired positions. Alternatively, the scanning element may be designed to scan the whole area of the sample, and the light-control element suppresses the generation of the light beam (e.g. by turning the light source off) or the propagation of the light beam (e.g. by closing a shutter) if the light beam would be directed to a position within the sample-ROI.

According to another embodiment of the invention, the abovementioned light-control element may be adapted to receive positional data defining the sample-ROI. This allows for a flexible application of the sample isolation unit, as only the appropriate data have to be transmitted in order to make it isolate any desired shape of sample-ROI. In particular, it thus becomes possible that a different, individual sample-ROI is isolated from each sample.

Other embodiments of the invention comprise at least one of the following features:
- An image of the sample-ROI and/or of the remaining sample is generated after isolation of the sample-ROI.
- The sample-ROI is transferred from a carrier to a separate holder.
- A molecular assay is executed with the isolated sample-ROI, said assay preferably comprising a PCR step, a sequencing step, and/or a micro-array hybridization.
- The sample-ROI corresponds to an "image-ROI" (region of interest) which has been selected from an image of the sample.

It was already mentioned that the sample isolation unit and the corresponding method may particularly be applied in a method according to the first aspect and an apparatus according to the second aspect of the invention. Further information about the sample isolation unit may therefore be taken from the description of this method and apparatus.

The disclosure further relates to the use of the apparatus or the sample isolation unit described above for molecular diagnostics, molecular pathology, in particular for oncology applications, biological sample analysis, chemical sample analysis, food analysis, and/or forensic analysis. Molecular diagnostics may for example be accomplished with the help of magnetic beads or fluorescent particles that are directly or indirectly attached to target molecules.

### BRIEF DESCRIPTION OF THE DRAWINGS

Aspects of the invention will be apparent from and elucidated with reference to the embodiments described hereinafter.

In the drawings:
Fig. 1 shows schematically the examination of a sample according to the present invention;
Fig. 2 shows schematically a sample isolation unit according to the present invention;
Fig. 3 and 4 show photos of sample-ROIs after ablation of undesired material.

Like reference numbers refer in the Figures to identical or similar components.

### DETAILED DESCRIPTION OF EMBODIMENTS

Pathology diagnostic investigation of patient material (tissue and cells) is the basis of many treatment decisions, in particular in oncology. Standard, thin slices from a biopsy are presented on microscope slides and stained according to certain protocols to visualize the morphology of the tissue. More recently in situ staining for disease-specific biomarkers is being developed for companion diagnostics of targeted drugs. Assessment may be done with a bright field microscope. Slides need to be stored after investigation for a long period as back-up in case the diagnosis needs to be re-assessed.

Digital pathology is a new technology in which the microscope is replaced by a digital scanner which scans the stained tissue sections automatically and stores the images in digital format with sufficient resolution and color rendering that the pathologist can do the same diagnosis from the digital image as he/she would do directly at the microscope. The latter means that the digital image can replace the physical slide. Digital images are stored instead of the slides. The original biopsy sample is of course always stored as well.

Next to the above forms of analysis, tissue and cell biopsies may also be investigated with molecular methods (abbreviated as "molecular diagnostics" or "MDX"), like q-PCR and sequencing. This so-called molecular pathology is increasing in importance with the advent of new molecular biomarkers. Often the pathologist decides based on the morphological information to run a molecular test to identify the biological characteristics of the cancer tissue for the right therapy choice. Since many molecular biomarkers cannot be quantified in situ on tissue - or at least not with the required precision - a separate molecular diagnostics test, like PCR or sequencing is carried out on a sample which is taken from the biopsy, in general from a coupe that has already been taken from the biopsy. This tissue section is processed by cell lysis before the measurement of DNA or mRNA markers. As a consequence the spatial information is lost.

Tumor tissues generally consist of many different cell types, not only cancer cells, and even the cancer cells can differ a great deal in molecular constitution in different areas of the tumor. The result of a molecular analysis will therefore depend on the exact composition of the tissue section which is used as sample for the molecular test. The more diluted the cancer cells are, the more insensitive and inconclusive the test result will be. In addition heterogeneity within the cancer cell population will also cause noise in the MDX assays, reducing sensitivity and specificity as well as reproducibility.

In the near future it will also become important to select from a slide with a cancer tissue slice other cell types, like for example specific types of immune cells; also from tissue slides from other diseases than cancer specific cell types will need to be selected to perform molecular diagnostics on.

With digital pathology there is currently no possibility to run a molecular test on a subsection of a biopsy coupe. Tests on full coupes have suboptimal precision and sensitivity due to dilution of the target cells with benign cells of different origin (e.g. endothelial, fibroblast and immune cells) and cancer cell heterogeneity. With conventional pathology it is not possible to mark the tissue sections for molecular analysis precisely since the sample for molecular testing must come from a new slide as the stained and inspected sample needs to be stored.

Though molecular diagnostics information is of increasing importance for the correct diagnosis of cancer (and other diseases), it is in practice not used frequently by pathologists. As explained above, the main problem is to define a representative, well defined sample from a tissue biopsy section for MDX testing. Manual selection is imprecise due to the heterogeneity of the tumor tissue (or other disease tissue) in general and an imprecise location of the tissue section with respect to the tumor (or other disease) location. The manual selection can create contamination, especially for PCR which amplifies even very low concentrations of contamination. Manual selection does not allow for a good annotation of the tissue selected for MDX. Computer-aided selection of tissue suffers from loss of reference between consecutive slides, since the selection cannot be made from the very same tissue section that was used for the in-situ staining, which is the basis for the selection.

Hence there is a need for a precise selection of sample material for molecular testing based on a pathology image.

In order to address this need, a new approach is proposed according to which a region of interest (ROI) in a sample is first selected from an image of the sample and then extracted in reality from the physical sample and subjected to a molecular assay.

In an exemplary embodiment of this approach, a tissue slide may be stained according to a certain clinical indication, e.g. with a HER2 immuno-histochemistry or immunofluorescent stain (IHC), or a combination of staining assays. The slide may then be scanned by a digital scanner, and the resulting image may be analyzed by a computer program to identify and indicate areas of common features. Those areas may be presented to the pathologist for confirmation and adaptation if necessary. From those areas a region of interest, called "sample-ROI", may be defined automatically or semi-automatically by a software program that represents the part of the sample that is selected for MDX testing. Typical parameters are annotated to that sample-ROI, like the average expression of HER2 in the given example, and the statistical distribution of the expression over the cells and the cell composition in that selection. The coordinates of that selected sample-ROI may be transmitted to a sample isolation unit that takes care of the physical selection of the sample for MDX. The selected "sample-ROI" may be transferred to a transfer device or directly a disposable that is used for MDX testing. The MDX testing may comprise sample preparation and molecular analysis, like qRT-PCR, qrt-PCR, sequencing or next gen sequencing, or micro-array hybridization, or a combination of these. The results of that analysis may finally be related to the information from the tissue selection algorithm and optionally be interpreted and presented to the pathologist together with the digital image of the tissue in which the sample-ROI that was selected for MDX is indicated as well.

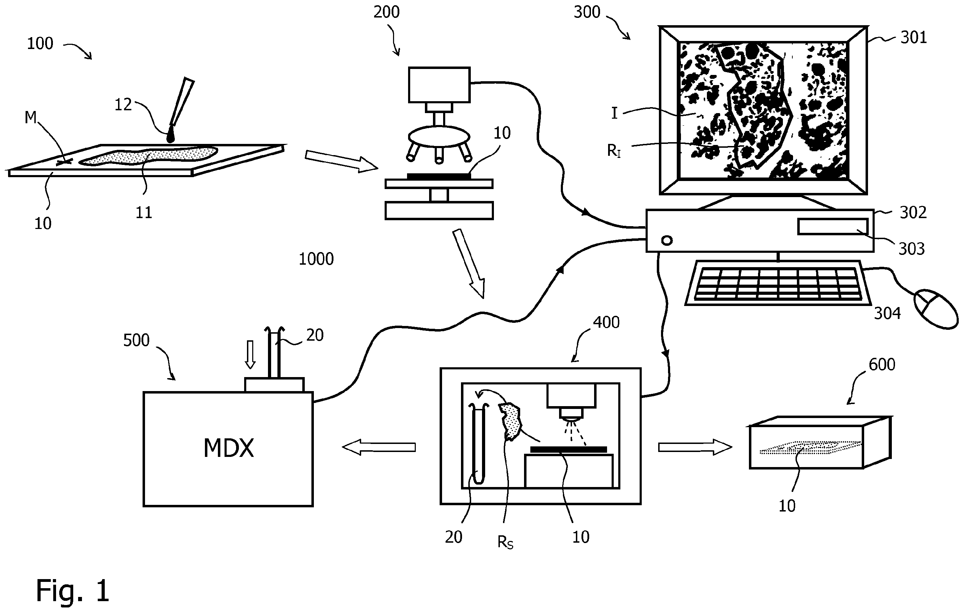

Figure 1 schematically illustrates an apparatus 1000 according to the invention that is suited for the examination of a sample 11 of body tissue according to the described procedure.

The examination starts at a sample preparation unit 100 in which a slice 11 of body tissue is prepared on a microscope slide 10 serving as a carrier. Typically, tissue samples are obtained by cutting a thin section of about 4-8 microns from a paraffinembedded biopsy. The so-called coupes are placed on the microscope glass slide 10 on a water film to relax from the micro-toming strain and are then left to dry.

Moreover, the sample 11 may optionally be stained by an appropriate stain 12, e.g. by Hematoxylin-Eosin (H&E) or IHC. There are a number of staining protocols available for different applications. Staining protocols can be carried out on the bench manually by dipping the slide with the coupe in different solutions containing the reagents, but can also be performed in an automated fashion.

One or more markers M may be printed on or engraved in the microscope slide 10 that can later serve as reference points for relating image coordinates to actual coordinates on the slide 10. Moreover, the slide 10 with the sample 11 is preferably covered with a cover slip (not shown) in order to allow for a high image quality during later scanning.

After the preparation step, the slide 10 with the sample 11 is transferred to an image generating unit 200, which may particularly be a digital scanning microscope.

A digital image I of the sample is generated by the microscope 200 and communicated to a sample selection unit 300, which is here realized by a workstation 302 with a display (monitor) 301, a memory 303, and input devices 304 like a keyboard and a mouse. The image I of the sample can be displayed on the monitor 301 to allow for a visual inspection by a pathologist. The pathologist can identify a region of interest, R_{I}, in the image and mark it accordingly.

The identification of this image-ROI RI may preferably be assisted (or completely be done) by automatic image analysis routines. As a first non limitative example, a software tool may identify the individual cells and calculate a score for the biomarkers in question based on the digital image of an IHC stained slide (optionally overlaid with information from a H&E scan). The tool may then calculate areas of similar or identical score with the respective statistics of cell numbers, average and histogram of scores of the cells in that area(s). The area can be optimized for total size, continuity and score statistics, as requested by the pathologist, optionally taking constraints into account which arise from the tissue selection technology. As a result of the calculation, an image-ROI RI is determined that may be visualized, together with the corresponding statistics, on the screen 301 and/or in the microscope 200 while looking at the sample slide. The image-ROI RI can then be adjusted manually and selected by the pathologist with the aid of a cursor.

As a second non limitative example, the identification of the image-ROI R_{I} may be defined in a first step by the pathologist making a coarse selection of an area inside or outside the image-ROI R_{I}. The area may for instance be identifed by the display of a border line including dots representing a certain number of clicks that the user made for quickly defining said area. In a second step, the area may be used in an algorithm such as the one just described in the previous paragraph for refining the positions of the borderline. In this effect the algorithm may notalby provide a coarse segmentation of said area to allow identification of the individual nuclei or cells. For each individual cell in the field-of-view features (e.g. stain uptake, cell type, cell proliferation, cell size, cell morphology, etc.) may be calculated. An adjustment of the area leading to a more accurate identification of the image-ROI RI may then be performed by seaching for neighbouring cells or nuclei with similar features as well-known in the art. Segmentation techniques as k-nearest neighbour or online machine learning can be used.

Preferably, the pathologist can mark positions in any magnification. The marking will typically be done based on tissue morphology, which is the basis for deciding on the malignancy of the lesion or on the different cell types present. An unlimited number of images can be selected and marked. When finished, the software may provide an overview of the selected image-ROIs in the appropriate magnification for the pathologist and adjust the areas to a necessary resolution which can later be processed by a sample isolation unit 400 which takes care of the physical selection/isolation of the sample for molecular testing. A file is created that contains the actual (image- and/or sample-) coordinates of the boundaries of the image-ROI RI (positive and negative selection) and the necessary references that can be interpreted by the sample isolation unit 400.

The aforementioned file can be used as input for either a device that can transfer this information to the slide, e.g. by a printing technology merely to indicate the areas which can then be removed manually or by another device, or as input for a sample isolation unit that is capable of removing the indicated sections directly and to transfer them to a sample holder that can be introduced into the molecular testing equipment (or sample prep equipment).

In the shown embodiment, the microscope slide 10 with the sample 11 is next transferred to the sample isolation unit 400 in which a sample-ROI, Rs, that corresponds to the selected image-ROI R_{I} is isolated (e.g. by a positive selection or a negative selection) and separated from the remainder of the sample. Preferably, this sample-ROI R_{S} is transferred to a separate holder, for example a test tube 20. Moreover, the sample isolation unit may preferably be capable to remove several areas consecutively and submit those to separate molecular tests. If the sample 11 on the slide 10 is covered with a cover slip, this will be removed before the isolation of the sample-ROI takes place.

The actual physical selection and transfer of tissue can be carried out in several ways. One of them is by laser microdissection (LMD). LMD is a technique which may be used to select individual cells or tissue parts from a tissue slide with the aid of a laserinduced transfer of cells to a tape or into a container. This technology allows the precise transfer of tissue. The LMD laser can move over the slide or alternatively the slide can move under a stationary laser beam. In the latter case all the sample will be collected at the same spot so that the collection device can be very compact.

In a digital pathology scan, the instrument moves a slide at hand underneath an objective lens. Tools may be used to find the area where the sample is present in order to optimize scanning time. The present application of such a scanning procedure requires having physical reference coordinates. Due to tolerances in slide dimensions it is preferred to have indicators on the slide, like the abovementioned mark M which can be read by the digital scanner simultaneously with the image scanning. An alternative is to use a mechanical stop and push the slide against the stop for reproducible positioning. Another alternative is to include a scanning function in the sample isolation unit that takes care of the selection and use software tools to overlay the newly scanned image and the original image with the depicted surface by feature recognition.

After isolation of the sample-ROI R_{S}, a new image may be scanned from the tissue slide 10 to confirm and control the selection. This image may be archived on the workstation 302 together with the original image and the results of the MDX analysis.

The test tube 20 with the sample-ROI R_{S} is in a final step transferred into a molecular examination unit 500 where assays of interest are executed with the sample-ROI. The microscope slide 10 with the remainder of the sample can optionally be stored in a storage unit 600 for later access and verification, or it may simply be discarded. Later processing steps with the slide 10 may particularly comprise a follow up examination comprising for example at least one new (particularly different) staining and/or at least one new (particularly different) molecular assay with another region of interest.

In the molecular examination unit 500, several molecular techniques may be available for analysis of the selected sample-ROI, like PCR (several techniques are comprised under this term, like q-PCR, RT-PCR, qrt-PCR, digital PCR, etc.) for detecting single point genetic mutations in cancer cells or any other DNA-mutation, DNA-deletion, DNA-insertion, DNA-rearrangements or copy number amplification or other structural change, and/or determining the degree of RNA expression of genes or other transcribed DNA sequences in cancer cells, or RNA or DNA sequencing (next gen sequencing) for determining a wider spectrum of genetic variations in the cancer cells, for example either on the whole genome, or the whole exome, or targeted to smaller regions of the genome, to the exosome, or the transcriptome, and in various depths. The result is interpreted in terms of genetic mutations of the cancer cells and their corresponding RNA expression profiles which are relevant for the prognosis or alternatively the susceptibility to certain treatment, like by targeted drugs, or to actually assess the effect of a treatment already started or finished (treatment monitoring). Moreover, the result of such a molecular analysis can be coupled to the image based analysis of the same sample section and reported together and also interpreted in combination.

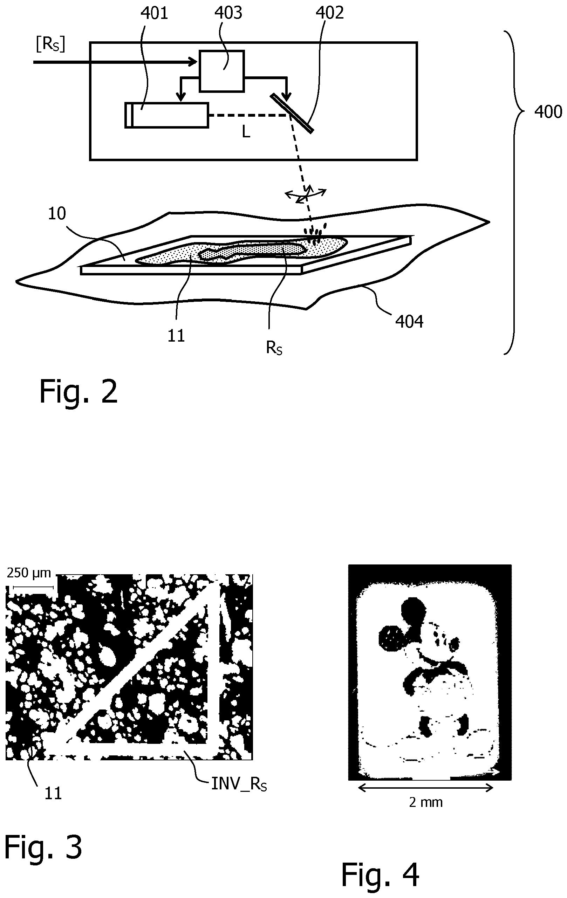

Figure 2 schematically shows a preferred sample isolation unit 400 according to the present invention. The sample isolation unit 400 generally allows for a new way of selecting tissue sample material from a thin section after histopathological investigation. The essential step of this approach is a removal of all material that should NOT be part of the sample-ROI, optionally followed by the total transfer of all remaining material into a test tube 20 for further analysis.

The removal of unwanted material may particularly be based on laser ablation. As explained above, the areas to be removed can be selected by a software tool based on pathological inspection following histopathological staining. Images of the remaining sample can be generated after removal of the unwanted material for precise documentation and characterization (e.g. quantification) of the input material for MDX analysis.

The particular sample isolation unit 400 shown in Figure 2 comprises a light source 401 by which for example a (laser) light beam L is generated. The light beam L is directed with a light directing system, for example comprising a scanning element like a movable mirror 402 and/or a shutter, onto the sample 11 provided on the slide 10. The light directing system further comprises a control unit 403 that receives data defining the desired sample-ROI on the slide 10 and that can switch the light source 401 on and off and/or control the movement of the scanning element 402. Thus the light source and/or the light directing system can be controlled in such a way that only positions outside the sample-ROI are irradiated by the light beam L.

In practice, a pulsed laser may be used with pulses having a power density of about 3 mW/µm². Typical wavelengths of the laser are e.g. 355 nm and 405 nm. With shorter wavelengths the absorption of tissue increases, but also that of the substrates in case one wants to operate in a closed compartment. The absorption of biological materials has a minimum in the green, unless the tissue is stained with labels that absorb in that range. If IR laser light is used, the absorption of water increases with wavelength. This plays a role because - depending on the device and assay - the tissue can be dry or saturated with water.

The negative selection, i.e. the removal of unwanted material, can be very fast as possible degeneration of the material is of no concern. A scanning laser or focused high power LED beam can be employed for the ablation of the material which is based on thermal effects due to absorption of radiation. The ablated material can condense or deposit on a waste depot or surface 404 that can optionally be replaced after each run.

The beam L of radiation may be controlled in the light directing system 402 by an actuator and a shutter and can scribe any pattern at a high resolution (determined by the components in the optical path). The remaining sample-ROI can be transferred into the test tube either by mechanical means or by a buffer solution. The procedure can be carried out in a closed or open system. The laser ablation can also be used to mark multiple areas that can then be selected manually separately.

The described approach is based on a scanning beam that removes material by thermal ablation. In contrast to laser microdissection, where the cells are transferred from the slide to a substrate or cup in a precisely controlled manner, here all material that is not wanted in the final sample is ablated so that only the selected sample material (sample-ROI) remains on the slide and the slide can be handled as if the whole section would be subjected to MDX. This means that the downstream procedure is independent of whether or not selection has taken place.

The light directing system 402 can be software controlled. The software control allows the use of user friendly visual interfaces that allow drawing of the selected areas on an interactive computer screen or equivalent tool, while zooming in or out, optionally combined with overlays of images from advanced staining, like IHC or ISH or any other method that supports the correct selection by the pathologist.

Depending on the light source used there may be enough power to expand the laser beam in one or two dimensions. This expansion can be modulated depending on the features of the surface that needs to be removed. Voids can be skipped. Standard slides can be used, but also dedicated substrates are possible to facilitate either the ablation process or the sample transfer to MDX or both. Moreover, the scanning procedure requires only a RELATIVE movement of light beam and sample and can hence also comprise an embodiment in which (additionally or alternatively) the sample holder 10 is moved, e.g. via a movable stage.

In an example a UV laser of 1 W at 355 nm was used to ablate FFPE tissue sample from a standard microscope slide. Figure 3 shows a photograph of the sample 11 after ablation of a triangular shape Inv_R_{S}. It can be seen that very clean areas can be removed with a sharp interface from the tissue section. One can conclude that the resolution for tissue removed is better than 50 micrometer at the chosen conditions. Tissue in between removed areas is stable, and the same resolution can be obtained with the tissue that stays behind. The used tissue was breast tissue at a thickness of 4 micrometers. The tissue was stained with H&E before laser ablation.

Figure 4 shows the results of the ablation of a larger area having a (negative) "Mickey Mouse" shape. The image demonstrates that also large areas can be removed readily and at any desired shape.

In summary, a method is described for selecting and annotating sample material for MDX analysis based on a histopathology staining. Histochemical scores on a cell to cell basis are used as selection criterion for determining a region of interest on the stained slide that represents the sample for MDX analysis. The selection can be done by an automated algorithm and/or optionally combined with manual adjustment by the pathologist. Alternatively, a coarse selection can be done manually by the pathologist combined with automatic adjustment by an algorithm. Statistical data may be created from that selection on cell types, numbers and scores. This information is linked to the MDX analysis. The MDX analysis uses only and precisely the tissue material of the described area. This is made possible by (i) using the same slide for (immune-) histochemistry and MDX sample selection, and (ii) having a digital image that contains all information so that the stained slide does not need to be kept intact and stored. The computer-aided selection of the region of interest is combined with an automated physical sample removal that interfaces with the MDX sample preparation and detection technology. The method results in a well-defined sample input for MDX. The input is taken into account with the evaluation of the MDX results. In this way MDX results can be annotated to cell types and histochemical scores of the same cells which make the pathological diagnosis much more precise and reproducible. By eliminating less relevant tissue, the precision and accuracy of these assays will improve leading to improved diagnosis. The approach is possible in combination with digital pathology scanners which can store the associated pathology image used for diagnostics, making storage of the actual slide for later referral superfluous. The overall procedure may comprise one or more of the steps:
1. Staining of a slide with a sample (e.g. IHC).
2. Digital scanning of the slide.
3. Storage of the resulting image file (e.g. PACS, IMS).
4. Interpretation of the image (e.g. by CAD).
5. Selection of areas ("image-ROI") for MDX (optionally using an optimization algorithm).
6. Annotation of areas for MDX (incl. scoring statistics).
7. Physical selection of areas ("sample-ROI") for MDX.
8. Scanning and storage of an image of the sample after selection.
9. Sample transfer to MDX holder(s) (cartridge, tube, ...).
10. Storage of slide with remainders.
11. MDX analysis of selected sample.
12. Annotation of MDX results with scoring statistics of image (from 6).
13. Interpretation of MDX (optionally using scoring statistics as input for interpretation algorithm).
14. Annotation of image with MDX results.
15. Interpretation of combined results of staining and MDX.
16. Potential new cycle of selection and MDX analysis.

According to another aspect of the invention, a method and device is described that allows a precise and efficient selection of sample material from tissue for further analysis, like qPCR and/or sequencing. The method is based on destroying/removing all sample material but the part that is selected for analysis (in contrast to the tedious positive selection that must render the selected material unaffected). This can be done very efficiently by laser illumination (e.g. scanning IR laser) that evaporates material at the exposed area. The remaining sample can easily be transferred into a test tube or cartridge for MDX analysis by hand or robotics. The method can be applied directly on the investigated tissue sections on a standard glass slide. Inspection after selection can be done readily since the selected material is unaltered and not displaced. High resolution is possible by a focused beam that is actuated with software control. High speed can be achieved depending on the laser power. The system can be linked to a digital pathology scanner and image analysis software for selection of the areas of interest and characterization of the input material.

An important advantage of the proposed approaches is the single tissue section, which implies a 100% accurate mapping of the selected image-ROI to the sample-ROI and a 100% complete annotation of the MDX sample. Moreover, more accurate and more reproducible MDX results can be achieved due to a quantified input and reduced heterogeneity of the MDX-sample, allowing for a better interpretation. The procedure also requires less handling (no manual steps) and less tissue (single coupe). It yields a digital file of staining and selection and MDX results in one, and the final interpretation includes a histopathological scoring of the selected MDX sample.

The invention may be applied in molecular pathology, in particular for oncology applications for patient stratification based on identification of molecular changes in cancer cells, but also for diagnostics/monitoring of other diseases.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure. The invention is defined by the claims. Any reference signs in the claims should not be construed as limiting the scope.

## Claims

1. A method for the examination of a sample (11), said method comprising the following steps:
a) generation of a digital image (I) of the sample (11);
b) analyzing said digital image with respect to at least one sample-parameter;
c) selection of a region of interest (Ri) in said digital image (I);
d) isolation of a region of interest (R_{S}) from the sample (11) that corresponds to the selected region of interest in said digital image;
e) execution of a molecular assay with the region of interest isolated from the sample to obtain assay-data;
f) linking assay-data to the sample-parameter of the corresponding sample region in order that the assay-data and said digital image are simultaneously accessible;
**characterized in that**:
the sample parameter indicates a local amount of a particular cell type or a tissue type.

2. An apparatus (1000) for the examination of a sample (11), comprising:
a) an image generating unit (200) configured for the generation of a digital image (I) of the sample (11);
b) an image analyzer configured for analyzing said image with respect to at least one sample-parameter;
c) an image selection unit (300) configured for the selection of a region of interest (R_{I}) in the digital image (I);
d) a sample isolation unit (400) configured for the isolation of a region of interest (R_{S}) from the sample (11) that corresponds to the selected region of interest in said digital image;
e) a molecular examination unit (500) configured for the execution of a molecular assay with the region of interest isolated from the sample;
f) an evaluation unit (301, 302, 303, 304) configured for linking assay-data to the sample-parameter of the corresponding sample region in order that the assay-data and said digital image are simultaneously accessible;
**characterized in that**:
the sample parameter indicates a local amount of a particular cell type or a tissue type.

3. The method according to claim 1,
**characterized in that** the sample (11) is stained before the generation of the digital image (I) and/or after the execution of the molecular assay.

4. The method according to claim 1,
**characterized in that** the sample (11) is covered by a cover slip during the generation of the digital image (I).

5. The apparatus (1000) according to claim 2,
**characterized in that** the image selection unit (300) comprises an automatic image analysis module and/or a user interface (301, 304).

6. The method according to claim 1 or the apparatus (1000) according to claim 2,
**characterized in that** the sample-parameter indicates the local amount of a particular cell-type or tissue-type.

7. The method according to claim 1 or the apparatus (1000) according to claim 2,
**characterized in that** the sample-parameter is at least partially based on a staining assay executed with the sample.

8. The method according to claim 1 or the apparatus (1000) according to claim 2,
**characterized in that** the selection of the region of interest (R_{I}) in said digital image is at least partially based on the sample-parameter.

9. The method according to claim 1 or the apparatus (1000) according to claim 2,
**characterized in that** the shape and/or size of the region of interest (R_{I}) in said digital image is adapted according to requirements set by regions of interest (R_{S}) which can possibly be isolated from the sample .

10. The apparatus (1000) according to claim 2,
**characterized in that** the sample isolation unit (400) comprises a laser microdissection device and/or a printing device.

11. The method according to claim 1 or the apparatus (1000) according to claim 2,
**characterized in that** the sample (11) is disposed on a carrier (10) which comprises at least one marker (M).

12. The method according to claim 1 or the apparatus (1000) according to claim 2,
**characterized in that** the executed assay comprises a PCR step, a sequencing step, and/or a micro-array hybridization, or another molecular diagnostic technique.

13. The method according to claim 1,
**characterized in that** the image selection comprises performing a manual coarse selection of an area in the image, and executing an algorithm to automatically adjust the area thereby obtaining a more accurate definition of the region of interest (R_{I}) in said image.

## Patentansprüche

1. Verfahren zur Untersuchung einer Probe (11), besagtes Verfahren umfasst die folgenden Schritte:
a) Erzeugung eines digitalen Bildes (I) der Probe (11);
b) Analysieren des besagten digitalen Bildes hinsichtlich wenigstens eines Parameters der Probe;
c) Auswahl einer Region von Interesse (R_{I}) im besagten digitalen Bild (I);
d) Isolierung einer Region von Interesse (Rs) in der Probe (11), die der ausgewählten Region von Interesse im besagten digitalen Bild entspricht;
e) Ausführung eines molekularen Versuches mit der Region von Interesse, die von der Probe isoliert wurde, um Versuchsdaten zu gewinnen;
f) Inbezugsetzung der Versuchsdaten zum Probenparameter der entsprechenden Probenregion, damit die Probendaten und das besagte Digitalbild gleichzeitig zugänglich sind; **dadurch gekennzeichnet, dass**:
der Probeparameter eine örtliche Menge eines besonderen Zelltyps oder Gewebetyps aufzeigt.

2. Ein Gerät (1000) für die Untersuchung einer Probe (11), umfassend:
a) eine Bildgebungseinheit (200), konfiguriert, um ein digitales Bild (I) der Probe (11) zu erzeugen;
b) einen Bild-Analysator, konfiguriert zur Analyse des Bildes hinsichtlich wenigstens eines Parameters der Probe;
c) eine Bildauswahleinheit (300), die zum Auswählen eines interessierenden Bereichs (R_{I}) in dem digitalen Bild (I) konfiguriert ist;
d) eine Probenisolationseinheit (400), die zum Isolieren eines interessierenden Bereichs (R_{S}) aus der Probe (11) konfiguriert ist, der dem ausgewählten interessierenden Bereich in dem digitalen Bild entspricht;
e) eine molekulare Untersuchungseinheit (500), die für die Ausführung eines molekularen Versuchs konfiguriert ist, wobei der interessierende Bereich aus der Probe isoliert ist;
f) eine Auswertungseinheit (301, 302, 303, 304), die zum Verknüpfen von Untersuchungsdaten mit den Probenparametern des entsprechenden Probenbereichs konfiguriert ist, damit die Untersuchungsdaten und das digitale Bild gleichzeitig zugänglich sind;
**dadurch gekennzeichnet, dass** der Probeparameter eine örtliche Menge eines besonderen Zelltyps oder Gewebetyps aufzeigt.

3. Verfahren nach Anspruch 1,
**dadurch gekennzeichnet, dass** die Probe (11) vor der Erzeugung des digitalen Bildes (I) und/oder nach Durchführung des molekularen Versuches angefärbt wird.

4. Verfahren nach Anspruch 1, **dadurch gekennzeichnet, dass** die Probe (11) während der Erzeugung des digitalen Bildes durch eine Abdeckung (I) abgedeckt ist.

5. Gerät (1000) nach Anspruch 2, **dadurch gekennzeichnet, dass** die Bildauswahleinheit (300) ein automatisches Bildanalysemodul und/oder eine Benutzerschnittstelle (301, 304) umfasst.

6. Verfahren nach Anspruch 1 oder Gerät (1000) nach Anspruch 2, **dadurch gekennzeichnet, dass** der Probeparameter die örtliche Menge eines besonderen Zelltyps oder Gewebetyps aufzeigt.

7. Verfahren nach Anspruch 1 oder Gerät (1000) nach Anspruch 2, **dadurch gekennzeichnet, dass** der Probeparameter wenigstens teilweise auf dem Versuch der Anfärbung basiert, der mit der Probe durchgeführt wurde.

8. Verfahren nach Anspruch 1 oder Gerät (1000) nach Anspruch 2, **dadurch gekennzeichnet, dass** die Auswahl der Region von Interesse (R_{I}) im besagten digitalen Bild wenigstens teilweise auf dem Probeparameter beruht.

9. Verfahren nach Anspruch 1 oder Gerät (1000) nach Anspruch 2, **dadurch gekennzeichnet, dass** die Form und/oder Größe des interessierenden Bereichs (R_{I}) in dem digitalen Bild entsprechend den Anforderungen angepasst wird, die durch interessierende Bereiche (Rs) festgelegt werden, die evtl. aus der Probe isoliert werden können.

10. Vorrichtung (1000) nach Anspruch 2, **dadurch gekennzeichnet, dass** die Probenisoliereinheit (400) eine Laser-Mikroschnittvorrichtung und/oder einen Drucker umfasst.

11. Verfahren nach Anspruch 1 oder Gerät (1000) nach Anspruch 2, **dadurch gekennzeichnet, dass** die Probe (11) auf einem Träger (10) angeordnet ist, der wenigstens einen Marker (M) umfasst.

12. Verfahren nach Anspruch 1 oder Gerät (1000) nach Anspruch 2, **dadurch gekennzeichnet, dass** der durchgeführte Versuch einen PCR-Schritt, einen Sequenzierungsschritt und/oder eine Mikroarray-Hybridisierung oder eine andere molekulardiagnostische Technik umfasst.

13. Verfahren nach Anspruch 1, **dadurch gekennzeichnet, dass** die Bildauswahl das Durchführen einer manuellen Grobauswahl eines Bereichs in dem Bild und das Ausführen eines Algorithmus zum automatischen Anpassen des Bereichs umfasst, wodurch eine genauere Definition des interessierenden Bereichs (R_{I}) im besagten Bild erreicht wird.

## Revendications

1. Une méthode pour l'examen d'un échantillon (11), ladite méthode comprend les
étapes suivantes:
a) la production d'une image numérique (I) de l'échantillon (11);
b) l'analyse de ladite image par rapport à un moins un paramètre d'échantillon;
c) la sélection d'une région d'intérêt (R_{I}) dans ladite image numérique (I);
d) l'isolation d'une région d'intérêt (Rs) d'un échantillon (11) qui correspond à la région d'intérêt sélectionnée dans ladite image numérique;
e) l'exécution d'une analyse moléculaire avec la région d'intérêt isolée de l'échantillon pour obtenir des données d'analyse;
f) la liaison des données d'analyses au paramètre d'échantillon de la région d'échantillon correspondante afin que les données d'analyses et ladite image numérique soient accessibles de manière simultanée;
**caractérisée par le fait que**:
le paramètre d'échantillon indique une quantité locale d'un type particulier de cellules ou d'un type tissulaire.

2. Un appareil (1000) pour l'examen d'un échantillon (11) comprend:
a) une unité de production d'image (200) configurée pour la production d'une image numérique (I) d'un échantillon (11) ;
b) un analyseur d'images configuré pour analyser ladite image par rapport à au moins un paramètre d'échantillon;
c) une unité de sélection d'images (300) configurées pour la sélection d'une région d'intérêt (R_{I}) dans l'image numérique (I);
d) une unité d'isolation d'échantillon (400) configurée pour l'isolation d'une région d'intérêt (Rs) à partir de l'échantillon (11) qui correspond à la région d'intérêt sélectionnée dans ladite image numérique;
e) une unité d'examen moléculaire (500) configurée pour l'exécution d'une analyse moléculaire avec la région d'intérêt isolée de l'échantillon;
f) une unité d'évaluation (301, 302, 303, 304) configurée pour lier des analyses de données au paramètre d'échantillon de la région d'échantillon correspondante afin que les analyses de données et ladite image numérique soient accessibles de manière simultanée; **caractérisée par le fait que**:
le paramètre d'échantillon indique une quantité locale d'un type particulier de cellules ou d'un type tissulaire.

3. La méthode selon la revendication 1,
**caractérisée par le fait que** l'échantillon (11) est coloré avant la production de l'image numérique (I) et/ou après l'exécution des analyses moléculaires.

4. La méthode selon la revendication 1,
**caractérisée par le fait que** l'échantillon (11) est couvert par une lamelle couvre-objets lors de la production de l'image numérique (I).

5. L'appareil (1000) selon la revendication 2,
**caractérisé par le fait que** l'unité de capture d'image (300) comprend un module d'analyse d'image et/ou une interface utilisateur (301, 304).

6. La méthode selon la revendication 1 où l'appareil (1000) selon la revendication 2
est **caractérisé par le fait que** le paramètre d'échantillon indique la quantité locale d'un type particulier de cellules ou d'un type tissulaire.

7. La méthode selon la revendication 1 ou l'appareil (1000) selon la revendication 2,
est **caractérisée par le fait qu'**au moins un paramètre d'échantillon est fondé partiellement sur au moins un test de coloration exécuté avec l'échantillon.

8. La méthode selon la revendication 1 ou l'appareil (1000) selon la revendication 2,
est **caractérisé par le fait que** la sélection de la région d'intérêt (R_{I}) dans ladite image numérique qui est fondée partiellement sur au moins un paramètre d'échantillon.

9. La méthode selon la revendication 1 ou l'appareil (1000) selon la revendication 2
est **caractérisé par le fait que** la forme et/ou la taille de la région d'intérêt (R_{I}) dans ladite image numérique est adapté selon les exigences établies par les régions d'intérêts (R_{S}) qui peuvent être isolées de l'échantillon.

10. L'appareil (1000) selon la revendication 2, **caractérisé par le fait que** l'unité d'isolation (400) d'échantillon comprend un dispositif à microdissection laser et/ou un dispositif d'impression.

11. La méthode selon la revendication 1 où l'appareil (1000) selon la revendication 2,
est **caractérisé par le fait que** l'échantillon (11) soit disposé sur un support (10) qui comprend au moins un marqueur (M).

12. La méthode selon la revendication 1 où l'appareil (1000) selon la revendication 2,
est **caractérisé par le fait que** l'analyse exécutée comprend une étape PCR, une étape de séquençage, et/ou une hybridation de microréseaux, ou une autre technique de diagnostic moléculaire.

13. La méthode selon la revendication 1,
**caractérisée par le fait que** la sélection d'images comprend l'exécution une sélection manuelle approximative d'une zone dans l'image, et l'exécution d'un algorithme pour ajuster de manière automatique la zone obtenant ainsi une définition plus précise de la région d'intérêt (R_{I}) dans ladite image.
